Europäisches Patentamt

(19) European Patent Office

Office européen des brevets

(11) Publication number: **0 060 129**
**B1**

## EUROPEAN PATENT SPECIFICATION

(45) Date of publication of patent specification: **15.11.89**

(21) Application number: **82301181.2**

(22) Date of filing: **08.03.82**

(51) Int. Cl.⁴: **A 61 K 39/02,** A 61 K 39/108
// C12N15/00

(54) **Vaccines, a method for making vaccines and a method for stimulating antibody production.**

(30) Priority: **09.03.81 US 241594**

(43) Date of publication of application:
**15.09.82 Bulletin 82/37**

(45) Publication of the grant of the patent:
**15.11.89 Bulletin 89/46**

(84) Designated Contracting States:
**AT BE CH DE FR GB IT LI LU NL SE**

(56) References cited:
EP-A-0 001 930
DE-A-2 530 275
FR-A-2 282 908
FR-A-2 442 271

INFECTION AND IMMUNITY, vol. 21, no. 2,
August 1978, pages 405-411, American Society
for Microbiology, (USA). MAGDALENE SO et
al.: "Characterization of an Escherichia coli
plasmid encoding for synthesis of heat-labile
toxin: Molecular cloning of the toxin
determinant"

(73) Proprietor: **CETUS CORPORATION**
**1400 Fifty-Third Street**
**Emeryville California 94608 (US)**

(72) Inventor: **Dallas, Walter Southwick**
**139 Drummond Place**
**Cary North Carolina (US)**

(74) Representative: **Bizley, Richard Edward et al**
**BOULT, WADE & TENNANT 27 Furnival Street**
**London EC4A 1PQ (GB)**

(56) References cited:
JOURNAL OF BACTERIOLOGY, vol. 145, no. 2,
February 1981, pages 920-925. P. L. SHIPLEY et
al.: "Identification and cloning of the genetic
determinant that encodes for the K88ac
adherence antigen"

56 References cited:

JOURNAL OF BACTORIOLOGY.
PLASMIDS OF MEDICAL, ENVIRONMENTAL
AND COMMERCIAL IMPORTANCE, vol. 1, 26th-
28th April 1979, Editors K.N.Timmis and
A.Pühler, Developments in Genetics, pages
113-122, Elsevier/North-Holland Biomedical
Press, Amsterdam (NL): W.S. DALLAS et al.:
"The characterization of an Escherichia coli
plasmid determinant that encodes for the
production of a heat-labile exterotoxin"

JOURNAL OF BACTERIOLOGY, no. 139, no. 3,
September 1979, pages 850-858. W. S. DALLAS
et al.: "Cistrons encoding Escherichia coli
heat-labile toxin"

MICROBIOLOGY-1979, Edited by
D.Schlessinger, pages 233-236, American
Society for Microbiology, Washington D.C.
(USA). W. S. DALLAS et al.: "Characterization
of the Escherichia coli heat-labile toxin gene"

CHEMICAL ABSTRACTS, vol. 93, 1980, pages
439, no. 234917b, Columbus Ohio (USA). S. L.
MOSELAY et al.: "Nucleotide sequence
homology between the heat-labile enterotoxin
gene of Escherichia coli and Vibrio cholerae
deoxyribonuelein acid" & J: BACTERIOL: 1980,
144(1), 444-6

METHODS IN ENZYMOLOGY, (RECOMBINANT
DNA), vol. 68, 1979, pages 245-267. F. BOLIVAR
et al.: "Plasmids of Escherichia coli as cloning
vectors"

NATURE, vol. 242, 20th April 1973, pages
531,532. J.M. RUTTER et al.: "Protection
against enteric disease caused by Escherichia
coli - a model for vaccination with a virulence
determinant"

Nature 288, 499 (1980)
Infection Immunity, 29, 1125-33, 1980

## Description

This invention relates generally to the field of immunology and, more particularly, to an improved vaccine for prevention of gastro-enteric disease. The invention also relates to an improved method of stimulating antibody production in a mammalian species using such a vaccine and to an improved method for producing such a vaccine.

Many gastro-enteric diseases in humans and animals, such as those caused by *Escherichia coli* and similar bacteria, are generally the result of toxin production which operates to disrupt the fluid balance in the gastro-intestinal tract. The result is excessive production of fluids and electrolytes from the epithelial cells in the gastro-intestinal tract. (Moon, H. W., *Adv. Vet. Sci. and Compt. Med.*, 18: 179—211 (1974).) By way of example, certain strains of *E. coli* cause a cholera-like disease in humans and young farm animals which may result from the action of either of two toxins that have been isolated and identified as ST, which is heat stable, and LT, which is heat labile. Kohler, E. M., *Am. J. Vet. Res.* 29: 2263—2274, (1968); Gyles, C. L. & Barnum, D. A., *J. Inf. Dis.* 120:419—426 (1968).

Recent studies have shown that in order for pathogens to successfully colonize parts of the body, it is necessary for the pathogen to have the ability to adhere to the cell surfaces so that it can multiply. After colonization, the pathogens produce agents, such as toxins, which cause the undesirable symptoms. *Science* 209: 1103—1106, Sept. 1980. The adhesins necessary for this adherence are typically structures called pili, which are thread-like projections on bacterial cell surfaces and are typically necessary if the pathogen is to cause disease.

It has been established that immunity to pathogens can be conferred by immunizing against the toxins which they produce; Dobrescu, L., and Huygelen, C., *Zpl. Det. Med. B,* 23: 79—88 (1976), and by immunizing against the adhesin factor, Jones, G. W., and Rutter, J. M., *Am. J. of Clinical Nutrition,* 27: 1441—1449, (Dec. 1974); Nagy, B., *Infect. Immun.,* 27: 21—24, (Jan. 1980). Vaccines conferring such immunities have been prepared with killed pathogens which themselves contained genes for producing the offending substances. In addition, vaccines have been prepared using the pure adhesin itself.

Preparation of vaccines in the ways referred to above leads to a number of serious problems. If the pathogen itself is used, the pathogen must be attenuated or killed in order to avoid causing the infection which the vaccination is designed to prevent. Of course, this requires a high degree of quality control in the manufacture of the vaccine to ensure proper attentuation or killing of the pathogen. Moreover, manufacturing vaccines of this type in this way necessitates providing growth conditions for large amounts of pathogens which, in the case of human diseases, are potential infectants of the human beings associated with the manufacturing process. Additionally, great care must be taken to insure that the live pathogens do not escape to the surrounding environment.

A further problem with many pathogens is that created by so-called capsular antigens. These polysaccharide layers cause alteration in the surface antigens exposed to potential antibody reaction. Consequently, a set of antibodies stimulated by one type of vaccine may be ineffective against the same pathogen with altered surface characteristics resulting from capsular antigens. This, as well as the other problems mentioned above, add cost and complexity to the manufacture of vaccines in this way.

Vaccines have, as mentioned above, been developed using purified pili which are capable of causing an antibody response in a vaccinated host. Morgan, R. L. Isaacson, R. E., Moon, H. W., and To, C. C. *Infect. Immun.* 22: 771—777 (Dec. 1978). However, purification of the desired substance can be an exceedingly difficult and expensive procedure, greatly increasing the cost of such vaccines.

An attempt to overcome these problems has been by the preparation of live vaccines comprised of Salmonella-coli hybrids produced by the conjugative transfer of a *Salmonella* surface antigen into *E. coli. See,* DE—A—2530275. A vaccine comprised of one of these hybrid strains was protective against *S. typhimurium* infection in mice.

Vaccines comprised of formalin-treated bacterial strains prepared by transfer of an adhesin gene or an enterotoxin gene into a bacterial strain of low pathogenicity have been described in FR—A—2282908. However, to obtain multi-valent vaccines, two or more of the formalin-treated bacterial strains must be mixed.

The present invention enables the provision of a vaccine comprising a non-pathogenic microorganism and employing toxoids and adhesins as the antigenic determinants without requiring purification and isolation of such substances.

The invention takes advantage of the newly emerging technology of recombinant DNA. By using such techniques, microorganisms can be genetically engineered which are harmless but which make a protein product which stimulates production of desired antibodies. The genes which are inserted into the host microorganism are inserted by means of plasmid cloning vectors. The genes are both genes for an adhesin necessary for adherence of the pathogenic microorganism being vaccinated against, and genes for toxoids of a toxin causative of the disease. The non-pathogenic host in which the genes have been inserted manufactures the proteins encoded by those genes, thus enabling the vaccinated subject to produce antibodies in response to such proteins. These antibodies will then confer immunity against all other microorganism species which manufacture those proteins including, of course, pathogenic species.

In the case of adhesins, there are four known K88 types, ab, ac, ad and ad(e). K88(ac) and K99 have been cloned successfully. The identification and cloning of the gene that encodes K88(ac), as well as its expression in *E. coli* minicells, was described by Shipley et al., *J. Bacteriology* 145: 920—925 (1981). The cloning and expression of a DNA fragment that encodes K99 was described by Van Embden et al., *Infection and Immunity* 29: 1125—1133 (1980). These adhesins are factors in neonatal diarrhea in piglets, calves and lambs. They have also been proven to be the important immunogen antigenically determinant in provoking the protective response. Jones, G. W. and Rutter, J. M., *Am. J. of Clin. Nutr.* 27: 1441—1449 (Dec 1974); Guinee, P. A. M., Veldkamp, J., and Jansen, W. H., *Infect. Immun.* 15: 676—678 (1977). The gene for the heat labile toxin Lt has been cloned, So, M., Dallas, W. S., Falkow, S., *Infect. Immun.*, 21: 405—411 (1978). Also the gene for one subunit of LT, LT—B, a non-deleterious part of the toxin, has been cloned. LT—B has been shown to be determinant in stimulating the antibody response to the toxin LT.

LT has been demonstrated to be very similar to the toxin responsible for human cholera, Clements, J. D., and Finkelstein, R. A., *Infect. Immun.* 22: 709—713 (1978); Gyles, C., *Infect. Immun.* 9: 564—569 (1974); Dallas, W. S.; and Falkow, S., *Nature* 288: 499—501. Thus, LT—B can be successfully employed in live non-pathogenic microorganisms as a vaccine for this disease as well as for many of the *E. coli* produced diseases.

The present invention thus provides a vaccine for prevention of gastroenteric disease caused in a mammalian species by a pathogenic microorganism, said vaccine comprising cells of non-pathogenic *E. coli* wherein said *E. coli* cells are each capable of expressing genes encoding an adhesin necessary for the pathogenicity of the pathogenic microorganism and a toxoid of a toxin causative of the disease, said expressible genes being inserted into said *E. coli* cells via recombinant plasmids and being non-indigenous components of said plasmids. In another aspect, the invention includes non-pathogenic *E. coli* cells suitable for use in a vaccine for prevention of gastroenteric disease caused in a mammalian species by a pathogenic microorganism, wherein said *E. coli* cells are each capable of expressing genes encoding an adhesin necessary for the pathogenicity of the pathogenic microorganism and a toxoid of a toxin causative of the disease, said expressible genes being inserted into said *E. coli* cells via recombinant plasmids and being non-indigenous components of said plasmids.

The invention also provides a method for making a vaccine against gastroenteric disease in a mammalian species by a pathogenic microorganism, which method comprises introducing into cells of non-pathogenic *E. coli* expressible genes encoding an adhesin necessary for the pathogenicity of the pathogenic microorganisms and a toxoid of a toxin causative of the disease, said expressible genes being non-indigenous components of one of more stable replicative plasmids.

The following Examples are given to illustrate the invention and to describe the transformation of hosts using various plasmids or mixtures of plasmids.

Example I

Diarrheal disease in piglets is often caused by *E. coli* that produce two types of enterotoxins, LT (heat labile toxin) and ST (heat stable toxin). Both of these toxins are plasmid mediated. Gyles, C., So, M., and Falkow, S. *J. Infect. Dis.* 130: 40—49 (1974). *E. coli* strains producing such toxins frequently have a surface antigen composed of identical subunits that form filamentous surface appendages or pili and which give the pathogenic strain its adhesive properties for the epithelial cells of the upper intestine. Jones, G. W. And Rutter, J. M. *Infect. Immun.* 6: 918—927 (1972). A form of this antigen, K88, exists in at least four serologically distinguishable varieties known as K88ab, K88ac, K88ad and K88ad(e).

The K88 gene (K88ac) was first cloned at the University of Washingtion in Seattle. Shipley, P. L., Dallas, W. S., Dougan, G., and Falkow, S. *Microbiology*, 1979 (American Society for Microbiology, p. 176—180). Although the specific technique has not been published, it is essentially a standard cloning technique.

To accomplish this, the plasmid pPS100, which is 90Kb in size, is cleaved with the restriction enzyme *Hind*III. A 7.8Kb DNA fragment, that includes the K88 gene, is inserted into the *Hind*III site in the plasmid pBR322, producing a plasmid 12.1Kb in size, designated pPS002. This plasmid retains a functional gene for resistance to ampicillin (Amp$^R$) which enables the direct selection for transformants of *E. coli* K—12 harboring the plasmid. These transformants are then screened for tetracycline sensitivity (Tet$^S$) due to the insertion of a DNA fragment in the *Hind*III site of pBR322 which inactivates the gene controlling resistance to tetracycline. Tet$^S$ transformants are then tested for K88 production using K88 antibody. A small (4.8kb) fragment cut by the restriction enzyme *Eco*RI may then be removed to make the K88 plasmids less of a handicap to the host cell.

In actually reducing this portion of the invention to practice, several positive clones resulted from the screening process. All these clones have the same *Hind*III fragment, all were identical in observable phenomena, and all synthesized approximately four times as much K88 antigen as a wild type pathogenic strain as determined by radioimmunoassay. Although the cloning vector existed in the host at 10—20 copies per cell, the production of K88 was lower than 10—20 times that of the wild type, probably due to as yet upappreciated physiological controls in the host.

## Example II

K99 is a similar adhesin or pili which is responsible for a certain amount of enteric disease in pigs, and for a relatively larger amount of enteric disease in lambs and calves. Protocols are used essentially identical to those followed in Example I in connection with K88. The plasmid used was designated pBR313 (Tet$^R$Amp$^R$) and selection was for Amp$^r$, Tet$^S$, and agglutionation with K99 antibody. The result was a plasmid pwD010 (15: 45Kb).

## Example III

There are two ways of producing the two pili factors or adhesins in a single organism. This Example deals with a situation in which a single organism contains two different plasmids, one of which contains the K88 gene and the other of which contains the K99 gene. The immediately following Example, Example IV, illustrates the technique utilizing a single plasmid containing both the K88 and K99 gene.

The plasmids constructed in Example I containing the K88 genes were designated pPS002. The plasmid of Example II, pWD010, containing the K99 gene was derived from the plasmid pBR313. Since the plasmid pPS002 containing the K88 genes was derived from the plasmid pBR322, both plasmids pPS002 and pWD010 belong to the same incompatibility group. Only plasmids which are members of different incompatibility groups can stably exist together. Accordingly, the K99 gene of pWD010 was cloned as a *Bam*H-I fragment into a tetracycline resistance (Tet$^R$) gene of a plasmid designated pACYC184, producing a plasmid designated pCTS3002 (10.65kB). This plasmid confers chloramphenicol resistance (CM$^R$), to cells and has lost the function of its Tet$^R$ gene (Tet$^S$), and produces K99. The plasmid thus created contains the K99 gene, but belongs to a different incompatibility group from that of the plasmid containing the K88 gene.

Transformation of *E. coli* K12 with these two plasmids resulted in cells which produced both K88 and K99, although instability was noted after a time.

## Example IV

This Example, as mentioned above, relates to the utilization of a single plasmid containing both the gene for K88 and the gene for K99. The composite plasmid, designated pWD600, was 22.2Kb in size and conferred chloramphenicol resistance (CM$^R$) and both K88 and K99 expression to the host cell. It was created by inserting the *Hind*III fragment including the K88 gene from pPS002 (12.1Kb Am$^R$, K88+) being inserted into plasmid pCTS3002 (10.65Kb, CM$^R$, K99+). *Hind*III treatment of plasmid pCTS3002 (10.65Kb, CM$^R$, K99+). *Hind*III treatment of plasmid pCTS3002 was followed by alkaline phosphate treatment to prevent the plasmid from recircularizing on itself in the presence of T4 DNA ligase. The composite plasmid produced both antigens as assessed by agglutination tests, but the level of antigen production was lower than with each gene in a separate bacterium. However, the plasmid was stable and production continued.

## Example V

The LT gene was cloned from the plasmid p307 as described by So, M., Dallas, W. S., and Falkow, S., *Infect. Immun.* 21:405—411 (1978). A further series of steps produces the plasmid EWD299 as described in Dallas, W. S., Gill, D. M., Falkow, S., *J. Bacteriol.* 139:850—858 (1979). The cistron that encodes for the B subunit of LT was then cloned individually into the expression vector pJJS500 by cleaving EWD299 with *Eco*RI and ligating this DNA to *Eco*RI cleaved pJJS500. A plasmid was identified which specified LT—B production but no LT—A production and was designated EWD1000. The LT—B cistron is being transcribed from the UV5 *lac* promoter in this plasmid.

Assays indicate 10—20% greater production of LT—B in the non-pathogenic strain compared to the pathogenic wild type strain.

It should be noted at this point that pJJS500 also is known to contain a gene for the surface antigen of Hepatitis B. This surface antigen, however, is not expressed and therefore will not be present in a final product. However, it may be desired to totally eliminate the gene for the Hepatitis B surface antigen. To do this, the plasmid pJJS500 is cut by the restriction enzyme *Hind*III and *Bam*H-I to remove all of the gene for L—TB as well as the gene for the UV5 *lac* promoter. This fragment is then inserted into the plasmid pPM31 by inserting at the *Hind*III-*Bam*H-I gap created after appropriate removal of this fragment from the pPM31. The resulting plasmid is Tet$^S$ (because part of the gene for tetracycline resistance is removed with the *Hind*III-*Bam*H-I fragment). The plasmid is also absent for the gene for the Hepatitis B antigen.

## Example VI

LT—B and K88 have been produced in the same host from the two different plasmids pPS002 and EWD1000, as created in Examples I and V above. These composite plasmids are of different compatibility groups and so are stable in the same host. The antigen production is similar to previously discussed results. The transformed strains may then be used live, or may be attenuated or killed by known vaccination preparation techniques, in a vaccine preparation. Use may be in accordance with any suitable known vaccination technique, but they may be administered to pregnant ewes, cows or sows at six weeks, and again at four weeks, prior to farrowing using a single subcutaneous injection each time.

In all of the foregoing Examples, it is desirable that genes for antibiotic resistance be eliminated from the plasmids utilized. It is preferable to not have resistant genes in vaccines and these may be replaced by genetic elements, conferring the ability on the microorganisms constitutive expression of enzymes necessary for lactose utilization, allowing easy identification of bacteria with the recombinant plasmids.

It may be seen, therefore, that the invention provides improved vaccines and an improved vaccination procedure in which live or attenuated or killed non-pathogenic microorganisms are used. Thus, other toxins produced by the microorganisms, or other disease-carrying factors, are not present in the vaccine. A vaccine which is live and which contains a suitable adhesin factor along with the non-toxic antigenic determinant for a gastroenteric toxin could permanently colonize in the gastrointestinal tract, conferring long lasting immunity.

By challenging a mammal with cells or a vaccine of the invention, antibodies are produced using the natural mechanisms of the mammalian body without risk to health. Such antibodies may, for example, be isolated from the blood of the treated mammal by conventional techniques. Such antibodies can be a useful product in their own right.

Various modifications of the invention in addition to those shown and described herein will become apparent to those skilled in the art from the foregoing description.

## Claims

1. A vaccine for prevention of gastroenteric disease caused in a mammalian species by a pathogenic microorganism, said vaccine comprising cells of non-pathogenic *E. coli* wherein said *E. coli* cells are each capable of expressing genes encoding an adhesin necessary for the pathogenicity of the pathogenic microorganism and a toxoid of a toxin causative of the disease, said expressible genes being inserted into said *E. coli* cells via recombinant plasmids and being non-indigenous components of said plasmids.

2. A vaccine as claimed in claim 1, wherein said plasmids are devoid of a gene or genes for antibiotic resistance.

3. A vaccine as claimed in claim 1 or claim 2, wherein the pathogenic microorganism is *E. coli* and the adhesin is selected from K88, K99 and 987P.

4. A vaccine as claimed in any one of claims 1 to 3, wherein the pathogenic microorganism is *E. coli* and the toxoid is LT—B.

5. A vaccine as claimed in claim 1 or claim 2, wherein the disease is cholera and the toxoid is LT—B.

6. Non-pathogenic E. coli cells suitable for use in a vaccine for prevention of gastroenteric disease caused in a mammalian species by a pathogenic microorganism, wherein said *E. coli* cells are each capable of expressing genes encoding an adhesin necessary for the pathogenicity of the pathogenic microorganism and a toxoid of a toxin causative of the disease, said expressible genes being inserted into said *E. coli* cells via recombinant plasmids and being non-indigenous components of said plasmids.

7. Cells as claimed in claim 6 further defined by the feature(s) of any one of claims 2 to 5.

8. A method for producing antibodies against pathogens associated with gastroenteric disease caused by a pathogenic microorganism other than in the treatment of the human or animal body by therapy, which method comprises administering to a member of a mammalian species cells of non-pathogenic *E. coli* wherein said *E. coli* cells are each capable of expressing genes encoding an adhesin necessary for the pathogenicity of the pathogenic microorganism and a toxoid of a toxin causative of the disease, said expressible genes being non-indigenous components of one or more stable replicative plasmids harboured by said *E. coli*.

9. A method as claimed in claim 8 further defined by the feature(s) of any one of claims 2 to 5.

10. Antibodies which have been produced by a mammal in response to the stimulus of challenging said mammal with a vaccine as claimed in any one of claims 1 to 5 or cells as claimed in claim 6 or claim 7.

## Patentansprüche

1. Impfstoff zur Vorbeugung einer durch einen pathogenen Mikroorganismus in einer Säugerart verursachten Gastroenteritis, wobei der Impfstoff nicht-pathogene E. coli-Zellen umfaßt, die in der Lage sind, Gene eines für die Pathogenität des pathogenen Mikroorganismus erforderlichen Adhäsins und eines Toxoids eines die Krankheit verursachenden Toxins zu exprimieren, und die exprimierbaren Gene in die E.coli-Zellen mittels rekombinanter Plasmide eingeschleust und nichtendogene Bestandteile der Plasmide sind.

2. Impfstoff nach Anspruch 1, wobei die Plasmide frei von einem oder mehreren Genen für Antibiotika-Resistenz sind.

3. Impfstoff nach Anspruch 1 oder Anspruch 2, wobei der pathogene Mikroorganismus E. coli ist und das Adhäsin K88, K99 oder 987P ist.

4. Impfstoff nach einem der Ansprüche 1 bis 3, wobei der pathogene Mikroorganismus E. coli und das Toxoid LT—B ist.

5. Impfstoff nach Anspruch 1 oder Anspruch 2, wobei die Krankheit Cholera und das Toxoid LT—B ist.

6. Nicht-pathogene E. coli-Zellen, die geeignet sind zur Verwendung in einem Impfstoff zur Vorbeugung einer durch einen pathogenen Mikroorganismus in einer Säugerart verursachten Gastroenteritis, wobei die E. coli-Zellen in der Lage sind, Gene eines für die Pathogenität des pathogenen Mikroorganismus erforderlichen Adhäsins und eines Toxoids eines die Krankheit verursachenden Toxins zu exprimieren, und die exprimierbaren Gene in die E.coli-Zellen mittels rekombinanter Plasmide eingeschleust und nichtendogene Bestandteile der Plasmide sind.

7. Zellen nach Anspruch 6, weiter definiert durch die Merkmale aus einem der Ansprüche 2 bis 5.

8. Verfahren zur Herstellung von Antikörpern gegen pathogene Mikroorganismen, die Gastroenteritis verursachen, wobei das Verfahren keine therapeutische Behandlung des menschlichen oder tierischen Körpers ist; dadurch gekennzeichnet, daß man nicht-pathogene E. coli-Zellen an eine Säugerart verabreicht, die in der Lage sind, Gene eines für die Pathogenität des pathogenen Mikroorganismus erforderlichen Adhäsins und eines Toxoids eines die Krankheit verursachenden Toxins zu exprimieren, wobei die exprimierbaren Gene nichtendogene Bestandteile von einem oder mehreren stabilen, in E. coli replizierbaren Plasmiden sind.

9. Verfahren nach Anspruch 8, weiter definiert durch die Merkmale nach einem der Ansprüche 2 bis 5.

10. Antikörper, die von einem Säuger als Antwort auf die Immunisierung des Säugers mit einem Impfstoff nach einem der Ansprüche 1 bis 5 oder Zellen nach Anspruch 6 oder Anspruch 7 hergestellt worden sind.

**Revendications**

1. Vaccin pour la prévention de maladie gastrointestinale provoquée chez une espèce mammalienne par un micro-organisme pathogène, ledit vaccin comprenant des cellules de E. coli non pathogène, dans lequel lesdites cellules de E. coli sont capables chacune d'exprimer des gènes codant pour une adhésine nécessaire à la pathogénicité du microorganisme pathogène et pour une toxoïde d'une toxine provoquant la maladie, lesdits gènes exprimables étant introduits dans lesdites cellules de E. coli par l'intermédiaire de plasmides recombinants et étant des composants étrangers auxdits plasmides.

2. Vaccin selon la revendiation 1, dans lequel lesdits plasmides sont exempts d'un gène ou de gènes codant pour la résistance à un antibiotique.

3. Vaccin selon la revendication 1 ou 2, dans lequel le micro-organisme pathogène est E. coli et l'adhésine est choisie parmi K88, K99 et 987P.

4. Vaccin selon l'une quelconque des revendications 1 à 3, dans lequel le micro-organisme pathogène est E. coli et la toxoïde est la LT—B.

5. Vaccin selon la revendication 1 ou 2, dans lequel la maladie est le choléra et la toxoïde est la LT—B.

6. Cellules de E. coli non pathogène, propres à être utilisées dans un vaccin pour la prévention de maladie gastro-intestinale provoquée chez une espèce mammalienne par un micro-organisme pathogène, lesdites cellules de E. coli étant capables chacune d'exprimer des gènes codant pour une adhésine nécessaire à la pathogénicité du micro-organisme pathogène et pour une toxoïde d'une toxine provoquant la maladie, lesdits gènes exprimables étant introduits dans lesdites cellules de E. coli par l'intermédiaire de plasmides recombinants et étant des composants étrangers auxdits plasmides.

7. Cellules selon la revendication 6, définie en outre par la(les) caractéristique(s) de l'une quelconque des revendications 2 à 5.

8. Procédé pour la production d'anticorps contre des agents pathogènes associés à une maladie gastro-intestinale provoquée par un micro-organisme pathogène autre que dans les traitement de l'organisme humain ou animal par thérapie, lequel procédé comprend l'administration à un membre d'une espèce mammalienne, de cellules de E. coli non pathogène, lesdites cellules de E. coli étant capables d'exprimer des gènes codant pour une adhésine nécessaire à la pathogénicité du micro-organisme pathogène et pour une toxoïde d'une toxine provoquant la maladie, lesdits gènes exprimables étant des composants étrangers d'un ou plusieurs plasmides répliquants stables hébergés par ledit E. coli.

9. Procédé selon la revendication 6, caractérisé en outre par la(les) caractéristique(s) de l'une quelconque des revendications 2 à 5.

10. Anticorps ayant été produits par un mammifère en réponse à la stimulation d'une provocation dudit mammifère par un vaccin selon l'une quelconque des revendications 1 à 5, ou par des cellules selon la revendication 6 ou 7.